Europäisches Patentamt

European Patent Office

Office européen des brevets

(19)

(11) Publication number : **0 494 509 A1**

(12) **EUROPEAN PATENT APPLICATION**

(21) Application number : **91311347.8**

(22) Date of filing : **05.12.91**

(51) Int. Cl.$^5$ : **G01N 33/53, G01N 33/542**

(30) Priority : **11.12.90 US 625820**

(43) Date of publication of application :
**15.07.92 Bulletin 92/29**

(84) Designated Contracting States :
**AT BE CH DE DK ES FR GB GR IT LI LU NL SE**

(71) Applicant : **ENZYMATICS, INC.**
**500 Enterprise Road**
**Horsham, Pennsylvania 19044 (US)**

(72) Inventor : **Palmer, John L.**
**139 W. Gravers Lane**
**Philadelphia 19118 (US)**

(74) Representative : **Bassett, Richard Simon et al**
**ERIC POTTER & CLARKSON St. Mary's Court**
**St. Mary's Gate**
**Nottingham NG1 1LE (GB)**

(54) Assay, employing analog to digital signaling, for the presence of an analyte in a sample in conjunction with a bindingcomponent, apparatus for performing said process, and a method of performing said assay.

(57) An assay, method of performing said assay, and apparatus, capable of generating an "on"/"off" response from a changing concentration of sample analyte, are described. The assay system comprises a high affinity binding component for a sample analyte and has an analyte dissociation constant substantially less than the concentration of analyte to be measured. Under initial conditions, wherein there is an absence of added analyte, a substantial number of binding sites must be empty. The concentration of empty binding sites determines the threshold analyte concentration, or "trigger point," for the "on"/"off" switch. Also required are a means for determining when excess analyte is present after the binding sites are substantially completely filled, wherein said means has an affinity for analyte which is substantially less than the binding component affinity for analyte.

Also described is a diagnostic apparatus, comprising a set of compartments, each with a different threshold analyte concentration. A signal is generated in each compartment only when the analyte concentration is greater than the preset threshold, allowing instrument-independent, accurate measurement of analyte concentration.

FIG. 3

ANTIBODY-ANALYTE x 10$^{-8}$ vs LOG [ANALYTE]

EP 0 494 509 A1

## BACKGROUND OF THE INVENTION

The present invention relates to assays for determining the concentration of an analyte in a liquid. This new method allows one to obtain accurate, quantitative information without the use of instruments or complex laboratory experiments. This invention especially relates to homogenous binding assays or assays that can be read without a phase separation step. This invention also relates to apparatus and methods for carrying out these homogenous binding assays.

Traditional assays employ analog to analog (A/A) measurement of a generated signal. In A/A measurement, the signal strength produced varies proportionally with the input signal. For example, in a competition immunoassay the amount of displaced ligand is sigmoidally related to the concentration of added molecule of interest, referred to as an analyte. This sigmoidal displacement curve is measured and used as a standard curve to determine analyte concentration.

This invention utilizes an analog to digital (A/D) assay to determine analyte concentration. In this type of assay, the response from different analyte concentrations (the analog input) determines the presence or absence of a signal (the digital output). In A/D measurement, the strength of a signal is generally not important and is typically not measured. Rather, only the presence or absence of a signal is important.

Previous examples, though not necessarily in the prior art, of analog to digital chemical switches have involved reduction-oxidation (redox) chemistry. See U.S. Patent 4,059,407 and U.S. Patent Applications Serial Nos. 924,414, filed December 16, 1986, and 075,817, filed July 20, 1987.

Additionally, U.S. Patent Application Serial No. 160,595, filed February 26, 1988, teaches the use of analog to digital measurement for electron transferases.

The present invention employs a non-redox analog to digital chemical switch in conjunction with techniques previously used in immunodiagnosis.

Numerous methods for conducting competitive binding assays are known in the art. These methods can be divided into two classes: assays conducted entirely in one liquid phase, commonly called homogenous binding assays; and assays which require the separation of a solid phase from a liquid phase, herein called multi phase assays. The traditional radioimmunoassay (RIA) is an example of a multi phase assay, as is the sandwich assay. In these assays, a binding component containing a signal producing species is bound to or converted into a solid phase (e.g., by precipitation). The solid phase is separated from the liquid phase which contains signal that is not bound to the binding component. The amount of bound signal is measured and used to determine analyte concentration. The complexity of this separation requires multiple reagents and washing steps and greatly adds to the requirement for expensive measuring equipment and operator training.

The complexity of the above assays has led to the development of homogeneous immunoassays, wherein all of the reagents are mixed in liquid solution and the result is directly read. See U.S. Patent 4,442,204, columns 5-9. Current homogeneous assays are competition assays, wherein a binding component which binds with a signal to form a complex is employed. The binding component can be an antibody, immunoprotein, enzyme, or similar compound which has a high affinity for analyte. The analyte can be any chemical or biological molecule, including proteins, metabolites, drugs and drug metabolites, environmental contaminants, organic molecules, or any similar molecule, that can be bound by a binding component as defined herein. The signal molecule is typically a molecule producing a measurable signal which is equivalently bound to the analyte in such a way that the signal has a measurably different activity when bound to the binding component than when free of the binding component. In a competition assay, the binding sites of the binding component are substantially completely filled with signal. Analyte is added to a concentration near the binding component-analyte dissociation constant and allowed to "compete" with signal for binding sites. Using instruments, signal activity is then measured and used to determine analyte concentration.

The relative simplicity of homogenous binding assays has led to much commercial development. For example, the TDx™ assay commercialized by Abbott uses a fluorescent molecule as a signal molecule. The signal molecule has a more rapid tumbling rate when free than when bound to the binding component. This difference in tumbling rate causes a difference in circularly polarized fluorescence, which is measured by a fluorescence polarimeter.

Another commercial homogenous assay is the EMIT™ test of Syva Diagnostics. In this test, the signal is an enzyme-analyte conjugate that has a different activity when bound to the binding component than when free. A spectrophotometer is used to measure the amount of enzyme activity in the sample and this value is utilized in the calculation of analyte concentration.

The CEDIA™ homogenous binding assay commercialized by Microgenics and described in Clin. Chem., 32, 1637-1641 (1986), uses a multistep signal producing system. A $\beta$-galactosidase subunit fragment is covalently coupled to analyte. This enzyme fragment is prohibited from self assembly with the complementary fragment when bound to the binding component. When the conjugate is free from the binding component, self assembly occurs, producing measurable β-galactosidase activity.

These and other homogenous binding assays are more fully described in U.S. Patent Nos. 4,340,688, 3,817,837, and 4,043,872. All of these homogenous immunoassays utilize analog to analog (A/A) measurement. The present invention teaches how the above described, and indeed all, homogenous binding assays can be converted to A/D measurement under certain specified conditions.

Prior to the present invention, immunoassays focused on increasing sensitivity. Thus, A/A signaling, wherein the strength of a signal is recorded, was employed. The sensitivity of traditional assays is increased when analyte-signal conjugate concentration is in a 1:1 ratio with binding component. At this ratio, a change in analyte concentration causes the greatest change in bound signal. All added analyte contributes in the signal displacement reaction. The measurement recorded is the amount of free signal present, rather than the presence or absence of signal.

In analog to digital measurement, the signal producing component is a fraction of the total concentration of binding component. The analyte concentration where A/D measurement flips from an "off" to an "on" response is referred to as the "threshold analyte concentration." In A/D measurement not all analyte produces signal. Specifically, all analyte molecules below the threshold concentration will not produce any signal. In this regard, A/D measurement gives up sensitivity in exchange for increased ease of measurement.

There exists a need in the art for an effective assay which accurately measures an analyte concentration in a sample without complicated procedures or machinery. The assay should be simple to perform and the results should be easily read. In addition, there exists a need in the art for an apparatus for carrying out this assay.

## SUMMARY OF THE INVENTION

This invention aids in fulfilling these needs in the art. In accordance with the present invention, a new assay which signals the presence of an analyte in a sample when the analyte concentration exceeds a predetermined threshold is disclosed.

The assay comprises a quantity of a binding component which binds to an analyte, the binding component having an analyte dissociation constant which is substantially less than the threshold analyte concentration. The binding component has a predetermined quantity of initially empty binding sites having no analyte bound thereto. Under these conditions, added analyte will substantially completely bind to the binding component up to the point where the initially empty binding sites are exhausted. If analyte is added beyond this point, it will either: i) exist in solution as free analyte, which can interact with other reagents to produce signal; or ii) displace the signal generating means which are bound to the binding component, provided such signal generating means were bound to the binding component under initial conditions. Other binding constants in the system should not be the same as the binding component-analyte affinity in A/D measurement. Should the assay system require that displaced signal generating means or free analyte bind to other reagents, the displaced signal or free analyte should do so with substantially less affinity than they exhibit to the binding component. The quantity of initially empty binding sites is chosen such that in the presence of the threshold analyte concentration, substantially all of the initially empty binding sites will be filled by substantially all of the analyte in the sample.

The assay system also comprises a means for generating a signal when the analyte concentration in the sample exceeds the threshold analyte concentration. The signal generating means has an affinity for analyte that is negligible or substantially less than the affinity of the binding component for analyte. The analyte will therefore first fill all of the available binding sites of the binding component. Only when the analyte concentration exceeds the threshold level will free analyte be available to generate a signal.

A process for carrying out the above assay is also provided. This invention further provides an apparatus for carrying out the above assays. The apparatus comprises a set of compartments, each compartment containing a binding component having a different quantity of initially empty binding sites and a signal generating means.

## DESCRIPTION OF THE FIGURES

Figure 1 illustrates the difference between A/D measurement and traditional homogeneous immunoassays. Figure 1(a) illustrates a traditional A/A homogeneous assay in which each added analyte molecule is converted into a measurable signal. Figure 1(b) illustrates A/D measurement in which a significant number of initially empty binding sites titrate added analyte, removing the analyte from the solution and preventing its reaction with the signal generating components.

Figure 2 illustrates the traditional binding curve of [Antibody-Analyte] v. Log [Analyte] for monoclonal antibody.

Figure 3 illustrates the A/D binding curve taken from embodiment 2 of this invention.

Figure 4 illustrates the plot of signal producing analyte-receptor versus total analyte concentration in the regions very near a preset threshold.

Figure 5 comprises Table 1, which illustrates sample calculations of A/D switch resolution as a function of $(K_1/K_2)$ when [receptor]/[antibody] = 1,000.

Figure 6 illustrates switch resolution and conjugate synthesis, in which random and defined synthesis pathways are depicted.

Figure 7 illustrates an antibody gradient measuring device.

Figure 8 corresponds to an analog simulation program to A/D embodiment 1.

Figure 9 corresponds to the Tutsim™ solutions to embodiment 2.

Figure 10 corresponds to the analog simulation program for embodiment 3.

Figures 11(a) and (b) illustrate signal strength as a function of $K_1/K_2$.

Figure 12 comprises Table 2, which illustrates Tutsim™ calculations of embodiment 2 of an antibody A/D switch.

## DESCRIPTION OF PREFERRED EMBODIMENTS

The term "binding component" comprises all molecules that bind with an analyte of interest to form a binding component-analyte complex. It includes enzymes, antibodies, immunoproteins, for example, IgG, IgM, etc., as well as fragments of said antibodies and proteins capable of binding to the analyte of interest, such as the Fab fragment of IgG. The term "antibody" includes whole antibodies and fragments thereof having binding ability. Recombinant proteins formed by gene fusion of light and heavy chain antibody regions are also included in the definition of "antibody."

An enzyme can be employed as a binding component, as long as the binding ability of the enzyme, as opposed to its catalytic ability, is the relevant activity. For example, an enzyme might bind one component of a two component reaction. In the absence of the second component, the binding will not result in any reaction.

Additional binding components which can be employed include validamycin as a binding component for potassium ion; ethylene glycol tetraacetic acid (EGTA) as a binding component for calcium ion; N,N,N',N'-ethylenediamine tetraacetic acid (EDTA) as a binding component for ferric ion and other divalent and trivalent metal ions; and crown ether, receptors, chelating agents, and other similar binding substances which bind with the analyte of interest to form a binding component-analyte complex.

In the present invention, the concentration of the binding component refers to the concentration of the binding sites of the binding component employed. In the case of binding molecules with multiple binding sites, for example IgG or IgM, the real concentration will be lower than the concentration of binding sites, as is readily apparent.

Specificity in binding is a desired feature of a binding component, but absolute specificity is not required. In general, the binding component should not demonstrate high affinity binding for any component of the analyzed sample fluid other than the analyte. Also, the binding component should not have a lower affinity binding for any component that is in vast molar excess over the analyte. Such a component would be in competition with the analyte for the binding sites of the binding component. Thus, EDTA would be an excellent binding component in a fluid that contained only ferric ion analyte. A sample fluid that contains ferric ion analyte along with magnesium and other divalent metal ions requires the use of a binding component with more specificity than EDTA.

This invention is generally limited to binding components that have determinable affinity constants. A binding component which possesses a range of affinity constants can normally be used in this invention only when the range of constants is narrow enough so that one can determine that the analyte concentration is greater than the binding component dissociation constant.

The term "analyte" refers to any chemical or biological molecule, including proteins, metabolites, drugs and drug metabolites, environmental contaminants, organic molecules, etc., that can be bound by a binding component as defined herein. When the term "binding component" is used to describe a chelating agentb an "analyte" refers to the ionic substance that is chelated.

The term "threshold analyte concentration" refers to the concentration of analyte where A/D measurement flips from an "off" to an "on" response. Below the threshold analyte concentrationb substantially no measurable signal is present, while signal is detected above the threshold analyte concentration. The threshold analyte concentration is typically set in the manufacture of a diagnostic apparatus.

For example, in one embodiment of this invention, the assay system comprises a set of compartments, each with a different threshold analyte concentration. In a preferred embodiment, the compartment thresholds are linearly related to one another as a function of distance along the apparatus. The distance traveled along the apparatus by the signal thus corresponds to the concentration of analyte. Any measurable change in response can act as a signal. A change in color, particularly from colorless to colored or vice versa is useful. If the "on" signal is production of color, a colored bar would result in the compartments where the concentration of analyte is above the threshold analyte concentration. The bar would terminate at the location where the concentration of analyte was equal to the threshold, a concentration that could be read off the apparatus. The apparatus is similar to a thermometer, wherein a temperature is determined by reading the distance that mercury, which acts as a signal, travels along the thermometer.

An affinity constant numerically defines the intrinsic tendency of a substance to combine with another substance. Generally, they are used when constants are compared to one another. The larger the number, the greater the affinity. Dissociation constants numerically define the intrinsic tendency of a complex to decompose into its subcomponents. They are generally used when the constant is compared to a concentration. The affinity and dissociation constants, Ka and Kd, respectively, are related by the equations:

$$\text{For} \qquad A \quad + \quad B \iff A\text{-}B$$

$$Ka \; = \; 1/Kd \; = \; [A\text{-}B]/([A][B])$$

Thus, these two constants are reciprocals of each other. For example, a solution containing $10^{-6}M$ analyte will quantitatively bind to binding component containing initially empty binding sites if the binding component has a dissociation constant substantially smaller than the analyte concentration, e.g., $10^{-10}M$ (i.e., an affinity constant of $10^{10}M^{-1}$). These constants are used interchangeably throughout the description of this invention.

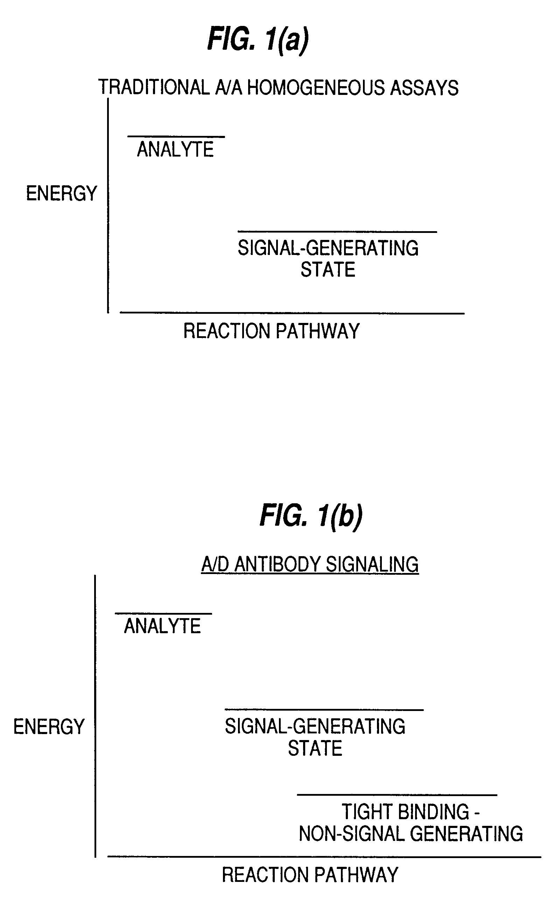

There are two clear distinctions between A/D measurement and traditional homogeneous immunoassays. The first difference is that in A/D measurement a significant concentration of binding sites are empty under initial conditions before analyte is added. These empty binding sites titrate added analyte, removing it from the solution and preventing its reaction with signal generating components. In contrast, traditional A/A homogeneous immunoassay, while using some of the same components as A/D measurement, is aimed at converting each added analyte molecule into a measurable signal. The difference in these two measurement forms is presented in Figure 1.

The difference in the graphs of Figure 1 results in the use of conditions that are distinctly different for A/D versus A/A measurement. Every molecule of analyte added to an A/A reaction will interact with the signal generating state, until the capacity to generate signal is exhausted. In contrast, in A/D measurement, added analyte will react with the tight binding - non-signal generating state (the empty binding sites) until such sites are filled (the threshold). Only after filling these empty sites will analyte react with the signal generating state. As is readily apparent, all current and future homogeneous A/A assays can be converted to an A/D assay in accordance with this invention by the addition of an appropriate tight binding - non-signal state.

Figure 1 graphically depicts the distinct differences between A/D and A/A measurement. As further described below, it is a critical feature of A/D measurement that all added analyte bind only to the empty binding sites until such sites are filled. This added analyte must bind essentially completely (as will be hereafter described) to such sites such that no free analyte is available to interact with the signal generating state.

In order to enhance the essential complete binding, it is important that the dissociation constant of the empty sites be considerably less than the concentration of analyte of interest. Indeed, this requirement for ultratight binding puts a limitation on the lower concentration of analyte that can be measured, as a physical limitation on antibody dissociation exists.

These low concentration limitations do not exist to the same extent for traditional homogenous immunoassays. Traditional homogeneous immunoassays, stripped of their apparent complexity, all fit the equation:

$$Ab - S + An \quad \xrightarrow{Ka} \quad Ab - An + S$$

Ab = antibody, An = analyte, S = signal producing system
As discussed above, the signal producing system, S, may generate signal directly or in combination with other components of the reaction system.

The reaction outlined in the equation above is defined by an apparent dissociation constant, Ka. The system is sensitive to changes in analyte concentrations only in regions near the Ka value. At analyte concentrations much lower than Ka, incremental changes in analyte concentration produce negligible changes in signal strength. At concentrations much higher than the Ka, again negligible changes in signal are produced in response to incremental changes in analyte concentration as essentially all signal is already free. In contrast, incremental changes in analyte concentration produce significant changes in signal strength when the analyte concentration is near the Ka concentration.

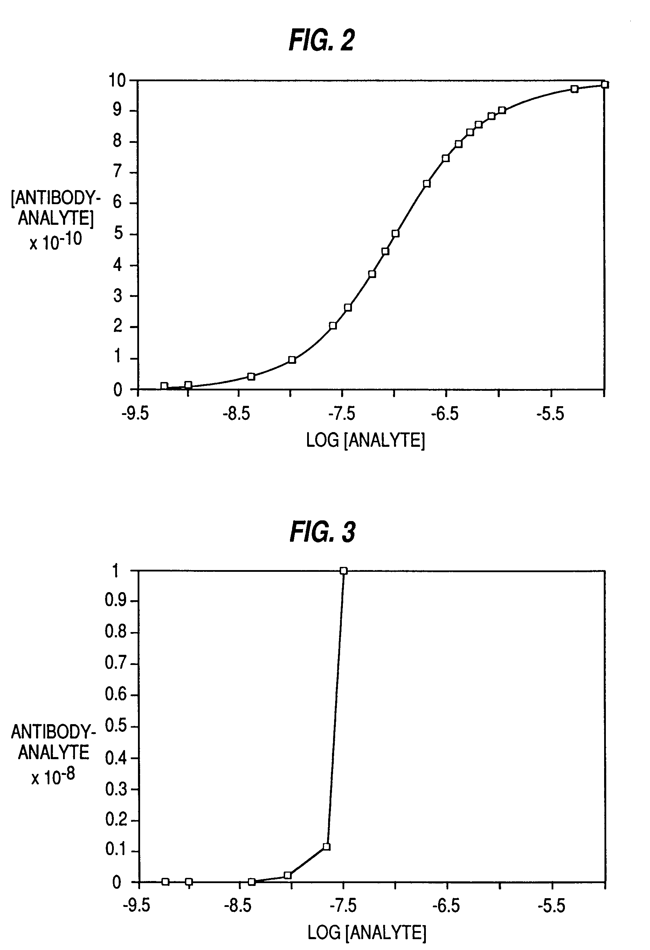

This situation is presented graphically in Figure 2. This figure demonstrates a typical binding component-analyte dissociation curve with a Ka of $10^{-7}$M. This graphic presentation confirms that incremental changes in analyte concentration produce significant changes in signal strength only in a range dispersed around the $10^{-7}$M dissociation constant, with a useful range of about 1.4 log units.

A typical A/D response is shown on this same log scale in Figure 3. A/D measurement requires the analyte concentration, at the threshold, to be substantially removed from the binding component-analyte dissociation constant. Note that while A/D measurement does not have a measurement range in the traditional sense, instead a sharp transition occurs between the logical zero, i.e., no signal state, and the logical 1, i.e., signal state. This transition is distant from the binding component-analyte dissociation constant, which is at -14 in the system depicted on this graph. These differences in the dissociation constant are a major distinguishing feature of A/D versus traditional A/A measurement. In A/A measurement, the useful measuring range is distributed around the systems analyte apparent dissociation constant. In A/D measurement, the measuring range is any analyte concentration significantly greater than the dissociation constant of the empty binding component binding sites.

Several embodiments of the invention are disclosed, including methods of adapting existing homogeneous assay technology to A/D measurement. The embodiments can be divided into the following three classes, in which, for purposes of illustration only and without limiting the lawful scope of this invention, the binding component is represented as an antibody:

Embodiment 1:

$$\text{antibody} + \text{analyte} \quad \xrightarrow{K_1} \quad \text{antibody-analyte (no signal)}$$

$$\text{analyte} + \text{receptor} \quad ===> \quad \text{analyte-receptor (signal)}$$

$$1/K_1 << [\text{analyte}]$$

Embodiment 2:

$$\text{antibody} + \text{analyte} \quad \xrightarrow{K_1} \quad \text{antibody-analyte (no signal)}$$

$$\text{analyte} + \text{antibody-signal} \quad ===> \quad \text{antibody-analyte} + \text{signal}$$

$$1/K_1 << [\text{analyte}] \text{ and}$$

$$[\text{antibody}] > [\text{antibody-signal}]$$

Embodiment 3:

$$\text{antibody + analyte} \quad \overset{K_1}{===>} \quad \text{antibody-analyte}$$

$$\text{antibody-cofactor + analyte} \quad ===> \quad \text{antibody-analyte + cofactor}$$

$$\text{factor + cofactor} \quad \overset{K_3}{===>} \quad \text{signal}$$

$$1/K_1 << [\text{analyte}], \quad [\text{antibody}] > [\text{antibody-cofactor}]$$

$$\text{and} \quad K_1 >> K_3$$

Embodiment 1 utilizes (1) a binding component that has a quantity of initially empty binding sites and which does not produce a signal upon binding with analyte and, (2) a signal generating means, or receptor, of substantially lower analyte binding affinity which produces a signal when bound to analyte. In this embodiment, a digital "on" signal is produced when, after analyte has substantially filled the initially empty binding sites, additional analyte interacts with receptor to produce a signal. Thus, the signal generating means will have analyte bound to it only when the analyte concentration is higher than the concentration of the initially empty binding component binding sites. In all other cases, the binding component will substantially quantitatively bind the analyte and prevent it from binding to the receptor. Examples 1 and 2 below illustrate embodiment 1.

Embodiments 2 and 3 are variations of embodiment 1. Embodiment 2 utilizes a signal means which produces a different response when bound to the binding component versus when it is unbound. As in embodiment 1, the binding component-analyte dissociation constant $(1/K_1)$ is substantially less than the threshold concentration. The initial conditions comprise a concentration of initially empty binding sites and a concentration of binding sites filled with a signal generating means. The concentration of initially empty binding sites determines the threshold analyte concentration. When the initially empty binding sites are substantially filled, additional analyte will displace signal from the binding component, producing a measurable response. Binding component displacement reactions, which are common to all homogeneous binding assay technologies, can be utilized in A/D measurement.

The third embodiment also comprises a signal produced by the displacement of a molecule that is initially bound to the binding component. This molecule is referred to as "cofactor." When displaced by analyte, free cofactor binds to a lower affinity component, called "factor," to produce a signal. The cofactor is initially present in a concentration less than the concentration of binding component binding sites. The remaining initially empty binding sites thus determine the threshold analyte concentration. In a preferred embodiment, the factor is an enzyme, while the cofactor is a prosthetic group, coenzyme, or regulatory component necessary for enzyme activity. This embodiment also requires that the binding component-analyte dissociation constant be significantly less than the threshold analyte concentration and that the affinity of factor for cofactor be less than the binding component cofactor affinity.

Embodiment 1

The reactions of embodiment 1 are defined by two equilibrium equations:

$$\text{Analyte + antibody} \quad \overset{K_1}{<===>} \quad \text{analyte-antibody}$$

$$\text{Analyte + receptor} \quad \overset{K_2}{<===>} \quad \text{analyte-receptor}$$

Analog to digital measurement is possible when an incoming signal, the analyte concentration, is partitioned between two different states which have a substantially different affinity for the analyte. In this situation, analyte will partition into the lower affinity state only when the higher affinity state is completely occupied.

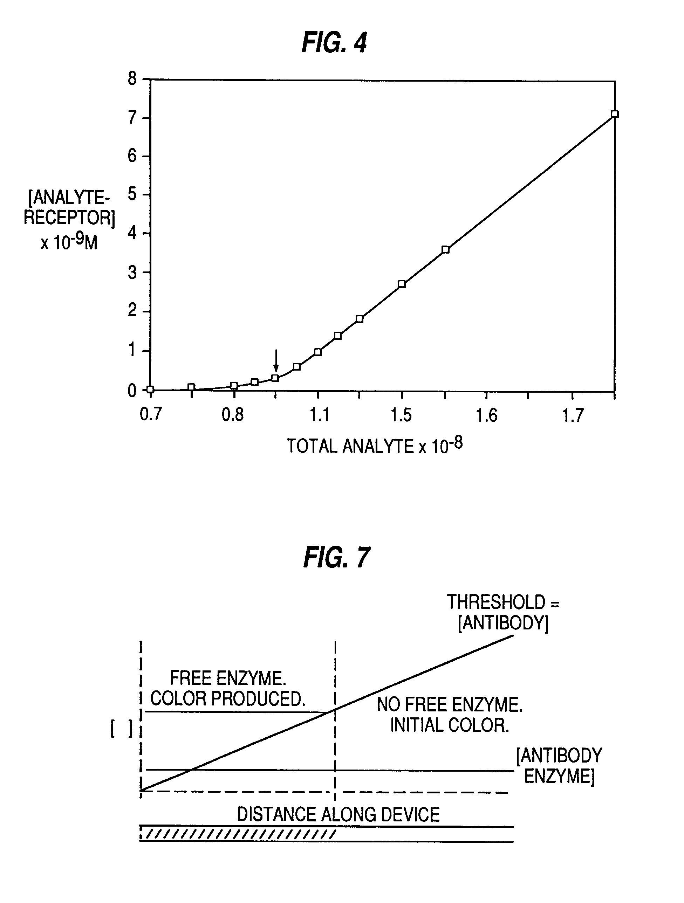

Figure 4 shows a plot of analyte-receptor concentration versus total concentration of analyte in regions adjacent to the threshold analyte concentration. Antibody concentration was set at $10^{-8}$M and receptor concentration was set at $10^{-5}$M. The $10^{-8}$M antibody concentration sets the threshold at $10^{-8}$M, a concentration less than the normal physiological concentration of most drugs and metabolic intermediates, and within the range of interest for measurement of hormones and other trace biologically active components. The antibody-analyte affinity constant, $K_1$, was $10^{12}$M$^{-1}$ and the receptor-analyte affinity constant, $K_2$, was $10^6$M$^{-1}$. This figure demonstrates a change in analyte-receptor concentration from essentially zero at concentrations less than the $1 \times 10^{-8}$M threshold analyte concentration to increasing concentrations of analyte above the threshold.

Figure 4 demonstrates a key feature of A/D switching: the analyte-receptor concentration rapidly increases from negligible values to the left of the threshold (logical 0) to detectable values to the right of the threshold (logical 1). The actual values in either state are not constant or important. In A/D signaling, the threshold concentration, where signal is rapidly increased from weak to strong, is important. The increase in analyte concentration required for this increase in signal is the switch resolution.

The number of initially empty binding sites of the binding component determines the threshold. As mentioned above, it is a critical feature of A/D measurement that the two states have a substantially different affinity for analyte. The ratio of the binding constants only poorly describes the relative affinity of the two states for an added analyte molecule. For example, a very high relative concentration of a lower binding state can have an overall affinity which is comparable to that of a low concentration of a high binding state. The relative affinity of analyte for these two states is proportional to the ratio of their binding constants and their concentrations. This relative affinity is best described by a partition ratio, defined as the product of the affinity constant for the binding component and the concentration of the binding component divided by the product of the affinity constant of the receptor and the concentration of the receptor. This is mathematically equal to:

$$\text{Partition Ratio} = \frac{K_1/K_2}{\text{receptor/antibody}} = \frac{[\text{antibody–analyte}]}{[\text{analyte–receptor}]}$$

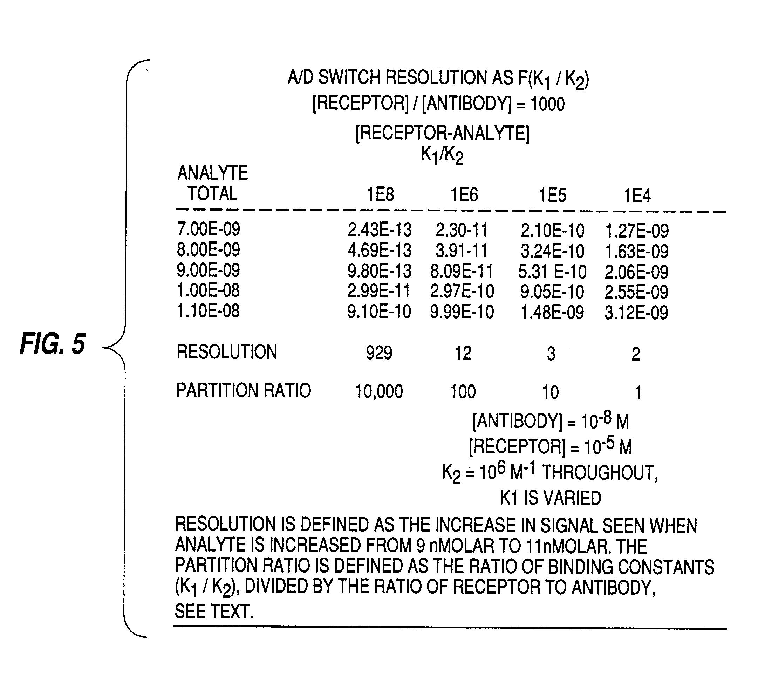

This equality derives from the mathematical definition of the binding constants, $K_1$ and $K_2$. Thus, this partition ratio represents the tendency for an added analyte molecule to form an antibody-analyte complex, rather than an analyte-receptor complex. The data presented in Table 1 of Figure 5 shows that the absolute values of the partition ratio are a much better predictor of switch resolution than the ratio of affinity constants. A partition ratio of 10,000 gave a switch resolution of over 900, and a partition ratio of 100 gave a switch resolution of 12, even though the affinity constant ratio varied by one hundred fold. A partition value of 10 yielded a poor resolution switch, while values less than 10 did not yield A/D behavior. The concept of the partition ratio is not well known in the art, although the realization that both a mass ratio and an equilibrium constant affect the extent of a reaction is well established. This simple ratio, which derives from the definitions of $K_1$ and $K_2$, was ideally suited to describe a critical feature of this embodiment, the partition of an added analyte molecule between the signal generating state, and a higher affinity non-signal generating state.

In A/D switching, added analyte binds to initially empty binding sites of the binding component, if any such sites are present. To ensure that this quantitative binding occurs, the binding component-analyte dissociation constant must be significantly less than the concentration of analyte at the threshold. This critical ratio is mathematically equal to:

$$\text{Binding Ratio} = (K_1) [\text{Threshold}].$$

$K_1$ = binding component-analyte affinity constant
[Threshold] = concentration of analyte at the threshold

A binding ratio of greater than or equal to 1,000 is needed for a high resolution switch. The higher this binding ratio, the greater the switch resolution. Thus, a ratio of 10,000 yields very high resolution, conversely, a ratio of 100 does not yield A/D behavior. This requirement for a binding ratio of about 1,000 or greater is the mathematical description of a distinguishing feature of A/D measurement discussed above. It is important that essentially all analyte, when present below the threshold, be tied up by binding component and not free in solution. This tight binding is achieved by this critical binding ratio. This requirement sets a practical lower limit of analyte concentration that can be measured with A/D switch technology at from about 1,000 to about 10,000 times higher than the dissociation constant of the antibody. Recent advances in monoclonal antibody production, particularly those methods that involve cloning of the antibody or Fab fragment and selection from bacterial sources, have made available antibodies of higher affinity than previously thought possible. It can now be contemplated that antibodies with affinity constants in excess of $10^{15}$M$^{-1}$ can be obtained. A high resolution Binding Ratio of 10,000 and a very high affinity antibody would bring A/D measurement to the $10^{-11}$ molar range, a concentration range lower than can be achieved by most conventional homogeneous immunoassays.

In summary, the data presented herein allows one to predict the conditions where A/D behavior will be observed. In this embodiment, an A/D switch will occur in all cases where both:

1) the partition ratio is at least about 10, prefer:sly at least about 100, and more preferably at least about 1,000.

2) the binding ratio is at least about 1,000, preferably greater than 1,000, and more preferably at least about 10,000.

These ratios represent preferred values and ratios outside of these values may also provide acceptable results.

The binding component in embodiment 1 is preferably an antibody having a high affinity constant for the analyte. The construction and selection of antibodies, particularly monoclonal antibodies, is well known in the art. Antibody fragments and genetically engineered antibodies are also well known to those skilled in the art and can also be employed where appropriate. Binding components other than monoclonal antibodies can also be readily used in this invention.

The signal generating means, or receptor, of embodiment 1 can be any compound that undergoes a change in catalytic activity, visible color absorbance, fluorescence, or a similar change upon interaction with the analyte. In particular, components that undergo a change in catalytic activity are useful, as such a receptor acts as a self-amplifying signal. For example, the signal generating means can be an enzyme that is stimulated or inhibited by the analyte. Given a digitoxin analyte, the receptor can be the F1 fragment of $Na^+K^+$-ATPase. As is well known in the art, digitoxin is a strong inhibitor of this ATPase. The reaction catalyzed by the ATPase, the conversion of ATP into ADP and $P_i$, will proceed normally in compartments where the concentration of binding component is in excess of digitoxin concentration. In compartments where the concentration of digitoxin is greater than binding component concentration, excess digitoxin will inhibit the ATPase and turn the reaction off. In this example, the "on/off" digital signal is the activity of the ATPase, and the analog input is the concentration of digitoxin.

## Embodiment 2

Embodiment 2 provides a second A/D assay system. While fundamentally similar to embodiment 1, embodiment 2 differs in the method of producing signal when the initially empty binding sites are filled. In embodiment 1, excess analyte binds to a receptor to produce a signal. In embodiments 2 and 3, excess analyte displaces a signal generating molecule that is already bound to the binding component. In embodiment 2, the displaced molecule directly produces a signal, while in embodiment 3, the displaced molecule is not active by itself but binds to another component to generate a signal.

Existing homogenous assay technologies can be applied to embodiment 2. For example, U.S. Patent 3,817,837 describes technology commercialized by Syva as EMIT™, wherein the signal is an enzyme. U.S. Patents 4,420,568, 4,492,762, 4,510,251, 4,585,862, 4,593,089, 4,748,110, 4,751,190, and 4,902,630 describe technology commercialized by Abbott Diagnostics as TDx™, wherein the signal generating molecule is a fluorescent molecule and the signal is a change in the polarization of laser induced fluorescence. These existing homogenous assay technologies employ traditional analog to analog measurement of a signal, rather than the analog to digital measurement of a signal employed in the present invention.

The reactions that occur in this embodiment are described by two equilibrium equations, which are similar, but not identical to the equations encountered in the previous embodiment.

$$\text{Analyte + antibody} \overset{K_1}{<===>} \text{analyte-antibody}$$

$$\text{Signal + antibody} \overset{K_2}{<===>} \text{signal-antibody}$$

Typically, the signal is a signal producing molecule covalently coupled to analyte in a manner such that the amount of signal produced is different between the bound and free state.

For ease of illustration, without limiting the lawful scope of the invention, the following discussion designates the signal of embodiment 2 as an enzyme analyte conjugate that is inhibited when bound to an antibody binding component and active when free.

Embodiment 2 requires a binding component with a high affinity for analyte and a signal generating means that binds to said binding component. This signal system has a change of activity when it is bound versus when it is unbound. Preferably, the signal system consists of an enzyme that has no catalytic activity when bound to the antibody. The enzyme expresses full catalytic activity when unbound from the antibody. The method of making such enzymes is well known in the art. See U.S. Patents 3,817,837 and 4,043,872. In general, analyte is covalently coupled to the enzyme adjacent to the enzyme active site. Alternatively, the enzyme is covered with multiple analyte molecules to cause sufficient antibody to bind and completely inhibit enzyme activity.

Three observations can be made regarding embodiment 2.

First, although A/D measurement requires an abundance of empty binding sites, the percentage of antibody sites occupied by enzyme is not critical over a wide range. The resolution of the A/D response is not affected by increasing the concentration of enzyme from 1% of the antibody concentration to 10% or even 50%, although the threshold is altered as the number of initially empty antibody sites is changed by the increase.

Second, it is advantageous for the antibody to have less affinity for the enzyme than for the analyte, resulting in a larger "on" signal. Without excluding other theories, this result can possibly be explained as follows. When the affinity constants are identical, excess analyte is not favored in competing with enzyme for binding sites. In contrast, when the affinity constants favor analyte by a factor of 100, excess analyte always wins the site competition.

Finally, the ratio of the antibody-enzyme dissociation constant ($1/K_2$) to enzyme concentration is important. Larger differences in this constant correspond to less noise in the switch. Switch "noise" is similar to static in radio reception of a stereo. This ratio effect is defined as the enzyme binding ratio:

$$\text{Enzyme binding ratio} = [\text{enzyme}]\,(K_2).$$

A ratio of approximately $10^3$ is required for very high resolution A/D signaling. Ratios significantly less than 100 do not provide A/D behavior of adequate resolution.

Again, without excluding other theories, this result can possibly be explained as follows. It is important that enzyme is completely bound by antibody until it is released and replaced by analyte which is in excess over empty binding sites. This quantitative binding requires an antibody-enzyme dissociation constant that is significantly less than the concentration of enzyme, just as quantitative binding of analyte requires an antibody-analyte dissociation constant that is significantly less than the threshold.

The following additional factors should be considered in preparing A/D assay systems according to embodiment 2.

First, the site competition cannot proceed faster than the enzyme "off" rate, wherein

$$K_2 = k_{on}/k_{off}$$

The "on" rate is the rate of binding of analyte to antibody and is generally found to be diffusion controlled. Diffusion controlled reactions have rate constants in the range of $10^9 M^{-1} sec^{-1}$. Thus, if we use this value for $k_{on}$, $k_{off}$ can be calculated at each equilibrium constant, $K_2$. This value of $k_{off}$ will control the rate of enzyme release from the antibody, which will control the rate of analyte replacing enzyme and generating signal.

The kinetic limitation of $k_{off}$ will put some practical limitations on the lower concentrations that can be measured using embodiments 2 and 3 of this invention. For example, to measure an analyte at a concentration of $10^{-10} M$, a concentration of enzyme of $10^{-10} M$ or less is required. The requirement for an enzyme binding ratio of greater than about 100 means that an antibody with a $K_2$ of greater than $10^{12} M^{-1}$ must be used. Thus, if $K_2$ is $10^{13} M^{-1}$ and kon is $10^{-9} M^{-1} sec^{-1}$, then $k_{off}$ is equal to $10^4 sec^{-1}$. The half time for a reaction is described by the equation:

$$T_{1/2} = \ln 2/k$$

Therefore, the $k_{off}$ calculated above will have a half time of 6,931 seconds, or about 2 hours. A reaction time of greater than about two half times is required for adequate measurement, which will take in excess of 4 hours. This reaction time compares favorably with that of conventional immunoassays, but is unacceptable for the simple, alternate site tests contemplated in part herein.

These kinetic limits do not appear when the analyte threshold and the enzyme concentration is set at 1 uM. An enzyme/antibody affinity constant, $K_2$, of $10^9 M^{-1}$ would give a high resolution enzyme binding ratio of 1,000. The off rate constant, $k_{off}$, under these conditions is 1 $sec^{-1}$, and the half time is 0.693 seconds. The above examples show that the off rate half time is linearly related to the log of $K_2$. When $K_2$ is $10^{10} M^{-1}$, the half time is about 6.93 seconds, and when $K_2$ is $10^{11} M^{-1}$, the half time is 69.3 seconds. An antibody with a $K_2$ of $10^{11} M^{-1}$ can, therefore, measure an analyte of $10^{-8} M$ under A/D conditions in less than 3 minutes.

The second factor to consider is the negative effect on switch resolution by any residual activity expressed by the enzyme-antibody complex. Residual activity is influenced by the character of the enzyme-analyte conjugation. Optimal results are obtained when analyte is linked to enzyme near enzyme active sites as opposed to random linking.

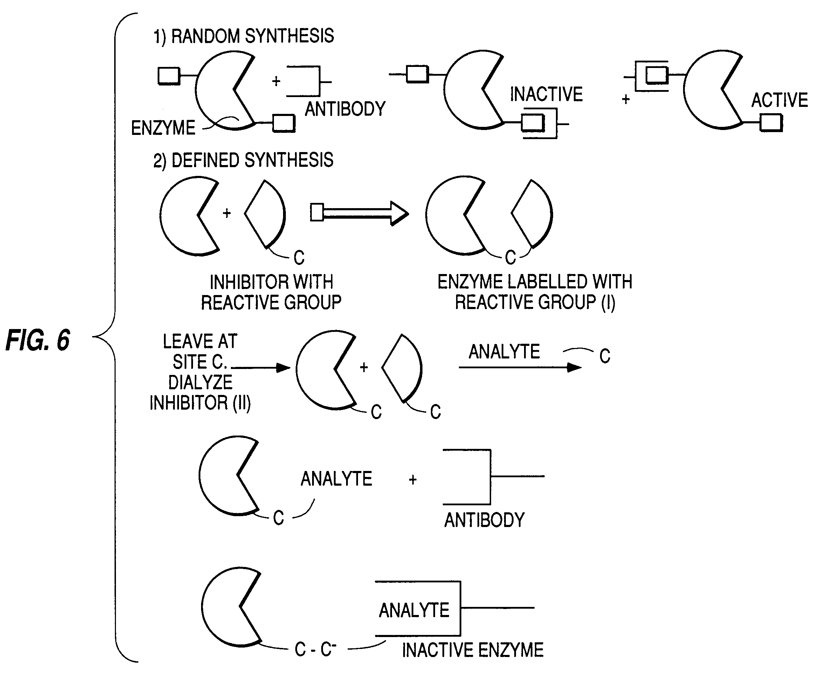

To reduce signal noise bound enzyme must be inactive. As illustrated in Figure 6, random synthesis of analyte-enzyme conjugate can result in linkage of analyte to regions removed from the enzyme active site. In some cases, antibody can bind to this conjugate without affecting the activity of the enzyme. The defined synthesis pathway in Figure 6 depicts a method for linking analyte close to the active site. An enzyme inhibitor is coupled to a protein modifying reactive group by a linker with a cleavable site, for example, a diol. The bound inhibitor-enzyme (I) is inactive. However, treatment with periodate and dialysis restores enzymatic activity. Analyte is then either directly, or via an additional linker, bound to the cleaved site. This procedure attaches analyte only near the enzyme active site. The resulting enzyme may disadvantageously have little or no activity. This can be cured by the use of linker arms of increasing length. Intermediate II should always be tested for activity. Inadequate activity of II can be solved by changing the reactive group or increasing the length of the reactive group to inhibitor linker.

In summary, A/D signaling for embodiment 2 results when the following conditions are met:

1) The binding ratio, as defined in embodiment 1, is at least about 1,000, preferably greater than 1,000, and more preferably, at least about 10,000;

2) The enzyme binding ratio is at least about 100, preferably at least about 1,000, and more preferably, at least about 10,000; and

3) The antibody concentration is greater than the concentration of enzyme, so that a number of empty binding sites will exist to set the threshold.

A/D signal strength is enhanced by:

1) Increasing the amount of enzyme relative to antibody. In Examples 1-3 below, switch resolution was not affected when enzyme was increased from 1% of antibody to 10% to 50%, while signal strength increased 100 fold over this range.

2) Increasing the ratio of the antibody-analyte affinity constant to the antibody-enzyme affinity constant.

Note that antibody affinity cannot be significantly reduced due to the requirements of point 2 above.

Example 2 below illustrates embodiment 2.

Embodiment 3

The following equations illustrate embodiment 3:

$$1) \quad \text{antibody} + \text{analyte} \quad \overset{K_1}{===>} \quad \text{antibody-analyte}$$

$$2) \quad \text{antibody} + \text{cofactor} \overset{K_2}{===>} \quad \text{antibody-cofactor}$$

$$3) \quad \text{factor} + \text{cofactor} \quad \overset{K_3}{===>} \quad \text{signal}$$

$$1/K_1 \; << \; [\text{analyte}] \; , \; [\text{antibody}] \; > \; [\text{antibody-cofactor}]$$
$$\text{and} \; K_1 \; >> \; K_3$$

Embodiment 3 employs an analyte-cofactor signaling system to produce an A/D response. When added analyte displaces cofactor from the binding component, the cofactor reacts with a factor to produce a measurable signal. The cofactor can be a coenzyme, such as FAD or NAD, while the factor can be an apoenzyme that lacks catalytic activity in the absence of the cofactor. Other stimulatory molecules can be utilized as cofactors in this embodiment, including enzyme allosteric effectors, hormones and other similar compounds. As discussed above, the cofactor and factor pair can also be self-assembling components of an enzyme monomer, wherein the individual components do not have independent separate activity. They can also be self-assembling pairs of molecules labeled with fluorescent molecules which either transfer energy or fluorescence when bound. Other such cofactor-factor pairs can be readily utilized in this embodiment. The only requirement is that the cofactor-factor combination produces a signal which is absent when the combination is separated.

The following observations are noted in regard to embodiment 3.

First, switch resolution is affected by the ratio of initially filled to initially empty antibody sites, or the ratio of antibody to cofactor. This is in contrast to embodiment 2, where this ratio has little or no effect on the resolution of the switch. A decrease in ratio from 1/100 to 1/10 lowered the switch resolution from 309 to 225 (resolution is defined as fold increase in signal per 10% change in analyte concentration) while increasing the signal strength (5) five fold. Further increase in ratio to 1/2 did not appreciably increase the signal strength while the effect on switch resolution was seriously disadvantageous. When utilizing this embodiment of the invention, one must choose carefully between increased signal strength and increased resolution, depending upon the needs of the particular application. This suggests that cofactor concentration should be as low as possible at the limit of where signal can be detected. This is a restatement of the general properties of A/D signaling: signal strength is reduced versus what would be observed under analog to analog conditions. A new potential for analysis is thus gained by this loss in strength.

Due to this loss of signal strength, self-amplifying signals, such as enzymes or other catalysts, are particularly useful in this invention. For example, if the factor is a FAD-analyte conjugate, and the cofactor is glucose oxidase apoenzyme, then the signal would be active glucose oxidase.

Second, the signal strength is increased as the ratio of $K_1$ to $K_2$ is increased. As in embodiment 2, once analyte has substantially filled the initially empty binding sites, it will compete with cofactor for binding component. If analyte has a stronger affinity for binding component than cofactor, analyte will "win" this competition and release a greater proportion of cofactor to produce signal.

Third, signal resolution is decreased (noise is increased) as the ratio of $K_2$ to $K_3$ is decreased. This was also observed in embodiment 1. The $K_2$ to $K_3$ ratio delineates the relative affinity of antibody versus enzyme for cofactor. A/D signaling requires that these affinities are substantially far apart. Thus, antibody should always "win" the competition for cofactor, as long as empty antibody binding sites exist. Holoenzyme should exist in this system only when analyte has displaced cofactor from antibody binding sites. A $K_2/K_3$ ratio of greater than about 1000 and more preferrably $10^4$ is advantageous for adequate switch resolution. The larger this ratio, the greater the resolution of the A/D switch.

Finally, as in embodiments 1 and 2, embodiment 3 also requires that the binding ratio is advantageously at least about 1,000, preferably greater than about 1,000, and more preferably, at least about 10,000. This binding ratio describes the ability of initially empty binding sites to tie up analyte. When testing is performed at ratios of 100 or less, the results tend toward traditional A/A measurement instead of the A/D measurement disclosed herein.

This embodiment shares a critical feature similar to the enzyme binding ratio of embodiment two; the cofactor binding ratio. This ratio is defined as the product of the affinity constant of the binding component for the cofactor and the concentration of the cofactor. This constant should be preferably greater than about 1,000, and more preferably greater than about 10,000. As in embodiment two, this critical ratio ensures that cofactor is completely bound to binding components until displaced by analyte. Example 3 below illustrates embodiment 3.

In embodiment 1, the low energy state is the binding component, while the high energy state that produces a signal is the receptor. The difference in analyte affinity constants between the low and high energy states describes the energy difference between these states. A substantial difference in energies (partition ratios) is needed to produce A/D signaling.

In summary, A/D measurement is possible in this embodiment when:

−the binding ratio is greater than or equal to about 1,000;

−the ratio of $K_2$ to $K_3$ is greater than or equal to about $10^4$;

−antibody exists in excess over cofactor to create a population of initially empty binding sites, the concentration of which defines the threshold;

−the cofactor binding ratio ($K_2$ * [cofactor]) is greater than or equal to about 1,000, and more preferably 10,000.

Switch resolution and signal strength are increased when:

−the ratio of initially empty antibody binding sites to sites filled with cofactor is kept as high as possible, preferably at a ratio of 9:1, and more preferably at a ratio of 99:1;

−the ratio of $K_1$ to $K_2$ is greater than 1, preferably 10 or greater.


## Summary

Traditional analog to analog measurement involves the interaction of an analyte with a signal producing state. Analog to digital measurement is possible anytime an analyte is partitioned between a low energy state that does not produce a signal and a higher energy state that does produce a signal.

In embodiments 2 and 3, the low energy state corresponds to the binding of analyte to initially empty binding sites, wherein the analyte has no competition. The high energy state comprises the binding of analyte to sites that are already occupied by signal.

Having generally described the invention, a more complete understanding can be obtained by reference to specific examples, which are provided herein for purposes of illustration only and are not intended to be limiting.

## EXAMPLES

### Example 1

To a series of test tubes is added increasing concentrations, from $5 \times 10^{-8}M$ to $5 \times 10^{-7}M$, of digitoxin antibody in isotonic phosphate buffer containing 5 mg/ml ovalbumin. The antibodydigitoxin binding constant has been previously measured at $4 \times 10^{13}M^{-1}$. Also added is $10^{-8}M$ of the $Na^+K^+$-ATPase F1 fragment. Digitoxin at $10^{-7}M$ is added to each test tube, followed by addition of ATP. ATPase activity is monitored and found in tubes that' have greater than $10^{-7}M$ antibody. Tubes with lesser antibody concentration have no activity, due to digitoxin inhibition of ATPase activity. ATPase activity can be measured employing any convenient method. A particularly useful method comprises coupling activity to color production as follows:

$$ATP \xrightarrow{\quad ATPase \quad} ADP + P_i$$

$$ADP + PEP \xrightarrow{\quad Pyruvate\ Kinase \quad} ATP + Pyruvate$$

$$Pyruvate + CoA + NAD^+ \xrightarrow{\quad Pyruvate\ Decarboxylase \quad}$$

$$Acetyl\text{-}CoA + CO_2 + NADH$$

$$NADH + Tetrazolium\ salt \xrightarrow{\quad Diaphorase \quad} NAD^+ + Formazan\ dye$$

### Example 2

Methotrexate (1 mM) was reacted with a 5 fold molar excess of 1-ethyl-3(3-dimethylaminopropyl)carbodiimide in $H_2O$. A solution of aminocaproic acid, adjusted to pH 8.5 in water, was added to a final concentration of 50 mM and the reaction was allowed to proceed overnight at room temperature. The derivatized methotrexate was purified by semi-preparative scale HPLC on a Cl8 reverse phase column run between 0.1% trifluoroacetic acid (TFA) in water and 80% methanol, 0.1% TFA. The purified drug was dissolved in water and lyophilized to dryness. The compound was again dissolved in $H_2O$ and reacted with excess carbodiimide. Trypsin (TPCK) treated in 1 mM HCl was added to the reaction and left to react at 4°C for one week. The reaction was dialyzed extensively against 1 mM HCl, 100 mM NaCl, and lyophilized to dryness. Analysis of the trypsin showed an average of 3.1 methotrexate linked per enzyme molecule.

A mouse monoclonal antibody, with a methotrexate binding constant of $1.5 \times 10^9M^{-1}$, was selected and shown to completely inhibit trypsin activity. Antibody-inhibited trypsin ($1 \times 10^{-6}M$) was mixed with 6% gelatin, 25 mM potassium phosphate, pH 7.8 buffer, and poured in a three thousands of an inch (mils) wet layer. This layer was coated with an antibody gradient layer of three mils wet thickness, containing $5 \times 10^{-6}$ to $5 \times 10^{-4}M$ antibody in 6% gelatin and 3% low molecular weight dextran. The film was dried, cut into strips 0.06 inches wide, and placed in the bottom of a rectangular plastic capillary with a film to plastic distance of 10.6 mils. The top of the capillary was previously coated with a gelatin solution containing N-p-tosyl-arg-p-nitroanilide, a chromogenic substrate for trypsin.

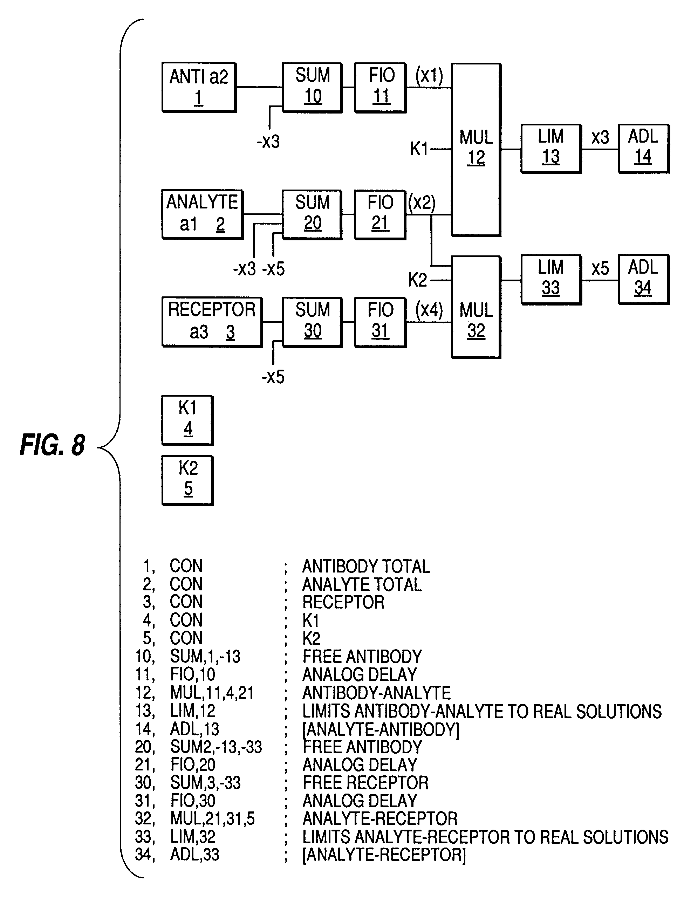

A serum solution is treated with trichloroacetic acid (5%) to precipitate proteins, then spun in a clinical centrifuge. The supernatant is removed, 10 mM of $K_3PO_4$ is added, and the solution is adjusted to pH 7 with $KHCO_3$. When this solution is introduced into the capillary, a colored bar develops within 5 minutes. The length of the colored bar is indicative of methotrexate concentration. The colored bar length is stable for greater than 2 hours and can be indefinitely stabilized by storage at -20°C. This device is graphically depicted in Figure 7.

### Example 3

The E. coli lac Z gene was engineered by recombinant DNA techniques, as delineated in *Clin. Chem.* 32, 1637-1641 (1986), to produce an enzyme acceptor and enzyme donor fragment. Digoxin was covalently linked to the enzyme donor fragment. A mouse monoclonal antibody with a digoxin affinity constant of $3 \times 10^{10}M^{-1}$ was obtained and shown to have an affinity for the digoxin-enzyme donor conjugate of $2 \times 10^9M^{-1}$. It was also shown that the conjugate had an affinity for the enzyme acceptor of $1.5 \times 10^4M^{-1}$. Increasing aliquots of a solution of antibody in gelatin were added to microtiter plates and dried under a gentle air stream. The antibody concentration (when hydrated in 100 µl) has a low point of 0.1 µM and a high point of 250 µM in 35 steps of increasing linear concentration. Prior to drying, conjugate was added to the antibody solution such that a constant ratio of 7.5% of the antibody sites were filled. A solution of 10 µM enzyme donor, containing 2 µM of chlorophenol red-β-D-galactopyranoside, was added to each well and again dried.

These plates can be stored at -20°C for over a year without loss of activity. They can be activated by addition of 100µl of an unknown solution of digoxin, with a concentration between 0.082 and 231 µM, containing 100 mM pH 7.4 phosphate buffer. The result is read after a 6 minute incubation. In wells where digoxin is present, concentration greater than the threshold will be dark red, whereas in tubes where initially empty antibody binding sites are present in excess over added digoxin, the initial color will remain. This assay can also be advantageously read by a microtiter plate reader coupled to an automated pipette for sample addition of the wells.

Mathematical Modeling

The invention also includes a method of mathematical modeling of the embodiments of this invention. This modeling is extremely useful in that the resolution of an A/D switch can be determined prior to experimentation once the binding constants of the components to be employed are known. This modeling is even more useful for predicting the necessary constants that must be obtained for A/D behavior in the development of new assays. This prior knowledge is of great assistance in the screening of monoclonal antibodies and other binding components.

The equations for the embodiments are:

## Embodiment 1

$$K_1 = [\text{antibody-analyte}]/([\text{antibody}][\text{analyte}])$$
$$K_2 = [\text{analyte-receptor}]/([\text{receptor}][\text{analyte}])$$
$$\text{Antibody total} = [\text{antibody}] + [\text{antibody-analyte}]$$
$$\text{Receptor total} = [\text{receptor}] + [\text{receptor-analyte}]$$
$$\text{Analyte total} = [\text{analyte}] + [\text{antibody-analyte}] + [\text{receptor-analyte}]$$

## Embodiment 2

$$K_1 = [\text{antibody-analyte}]/([\text{antibody}][\text{analyte}])$$
$$K_2 = [\text{antibody-signal}]/([\text{antibody}][\text{signal}])$$
$$\text{Antibody total} = [\text{antibody}] + [\text{antibody-analyte}] + [\text{antibody-signal}]$$
$$\text{Analyte total} = [\text{analyte}] + [\text{antibody-analyte}]$$
$$\text{Signal total} = [\text{signal}] + [\text{antibody-signal}]$$

## Embodiment 3

$$K_1 = [\text{antibody-analyte}]/([\text{antibody}][\text{analyte}]$$
$$K_2 = [\text{antibody-cofactor}]/([\text{antibody}][\text{cofactor}])$$
$$K_3 = [\text{cofactor-factor}]/([\text{cofactor}][\text{factor}])$$
$$\text{Antibody total} = [\text{antibody}] + [\text{antibody-analyte}] + [\text{antibody-cofactor}]$$
$$\text{Analyte total} = [\text{analyte}] + [\text{antibody-analyte}]$$
$$\text{Cofactor total} = [\text{cofactor}] + [\text{antibody-cofactor}] + [\text{cofactor-factor}]$$
$$\text{Factor total} = [\text{factor}] + [\text{cofactor-factor}]$$

Inspection of all three of these sets of simultaneous equations reveals that they contain cross terms. These cross terms convert the equation to simultaneous quadratic equations which cannot be solved by any known algebraic method. Most of the solutions to these equations require imaginary numbers. This lack of algebraic solution and extreme mathematical complexity has no doubt inhibited earlier appreciation of A/D measurement.

Solutions to these equations can be approximated by use of computer simulation techniques. Particularly useful is the simulation program Tutsim™, available from Tutsim Products, Inc., Palo Alto, CA. This simulation program converts a digital computer into an analog computer. An analog flow chart that mimics the equations listed above is constructed, with constant input blocks set at the concentrations of the total components and the binding constants. Time constant and limit blocks are added to this flow chart to prevent the computation from crashing and to force the solution to physically obtainable limits. The program is started and the output is sampled every 10,000 calculations. The calculations will settle down to a state of little or no change, indicating that equilibrium has been achieved. The calculated concentrations can then be tested in the above listed equations. The concentration of one parameter (e.g., the analyte concentration) can then be varied, and the calculations restarted to obtain the next equilibrium point. The Tutsim™ programs that fit embodiments 1-3 are listed in Figures 8-10.

Figure 8 corresponds to an analog simulation program to A/D embodiment 1. This program is run to find each point on the figures presented. The con blocks are set at the total concentration of analyte, antibody, and receptor, and at the binding constants. The sum blocks are used to obtain the concentration of free analyte, antibody, and receptor. The free component concentration is used in the mul blocks to calculate the concentration of analyte-antibody and analyte-receptor, the output of which is then fed back into the sum blocks. The flo blocks act as a time constant to prevent this closed loop system from crashing. The lim blocks limit intermediate concentrations to real values, and the adl blocks conveniently print out the data. The operating program, Tutsim™, was purchased from Tutsim Products, Palo Alto, CA. The program was run on a Dell 310, DOS computer fitted with a 386 compatible math coprocessor chip.

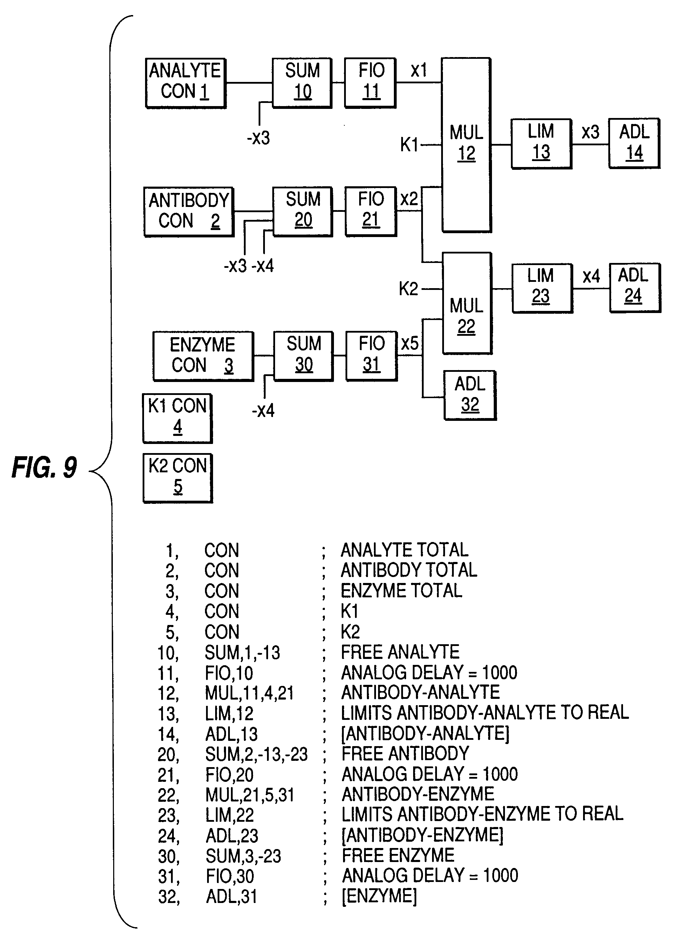

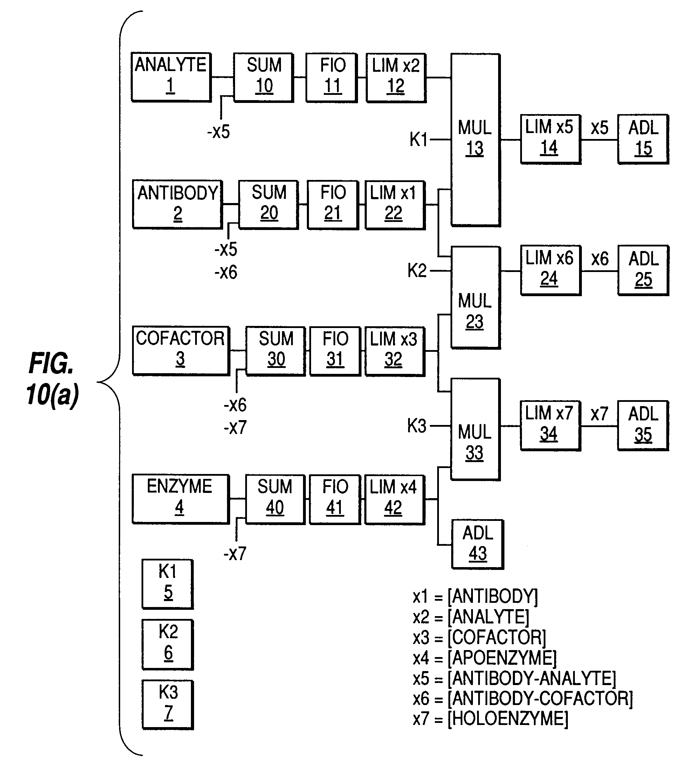

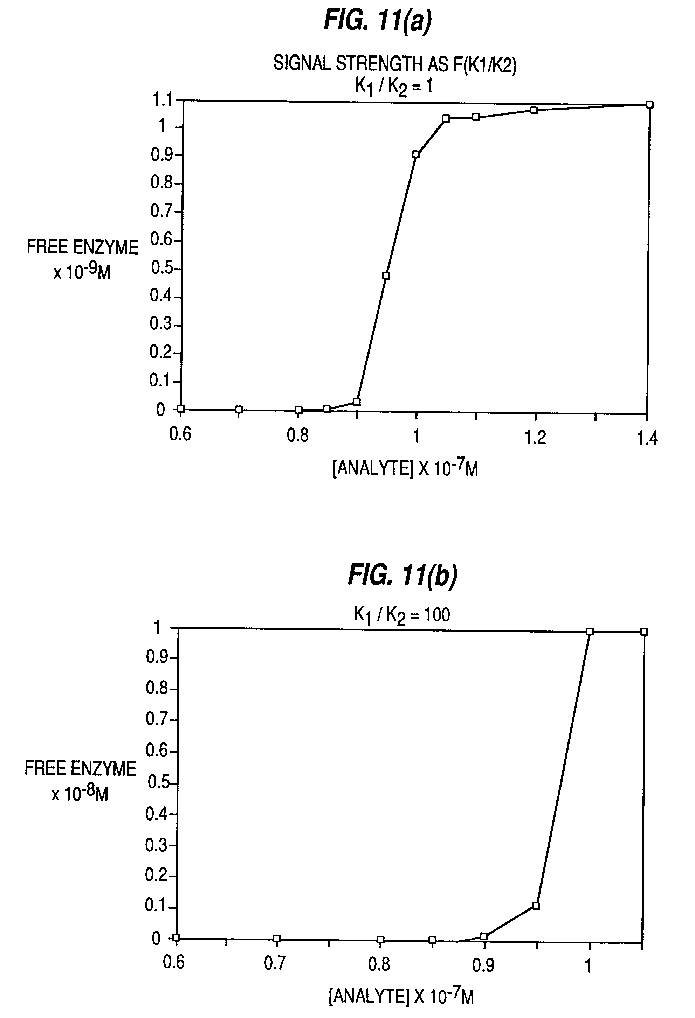

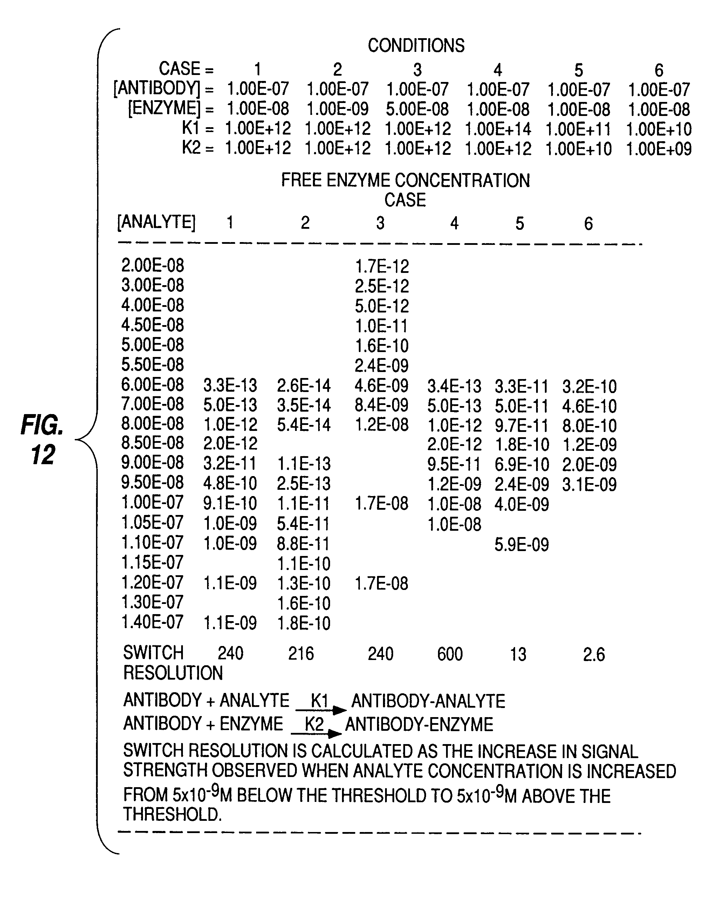

Figure 9 corresponds to Tutsim™ solutions to embodiment 2. The signal strength is investigated as a function of the affinity ratios, $K_1/K_2$. The results are shown in Figures 11(a) and (b) in which the threshold is set at $9x10^{-8}M$, and the total amount of enzyme is $10^{-8}M$. In Figure 11(a), both $K_1$ and $K_2$ are $10^{12}$, for a ratio of one (case 1 in Table 2 of Figure 12). Note that the signal at analyte concentrations greater than the threshold represents only about 10% of the available enzyme. In Figure 11(b), $K_1$ is increased to $10^{14}$, for a $K_1/K_2$ ratio of 100. The signal strength increases 10 fold; 100% of available enzyme is released. Cases 5 and 6 of Table 2 demonstrate that a $K_1/K_2$ ratio of 10 will increase the signal strength in intermediate amounts to approximately 40% of available enzyme.

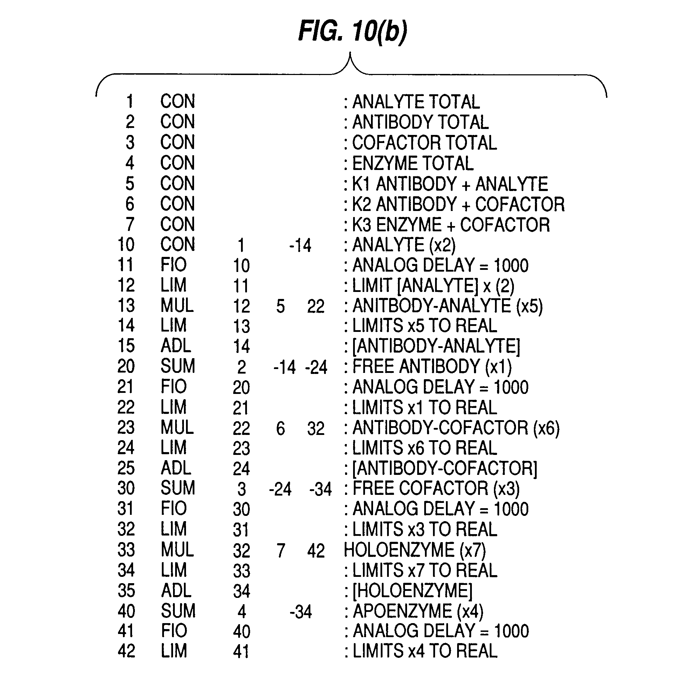

Figure 10 corresponds to an analog simulation program for embodiment 3. This program was run as indicated in the description of Figure 8, except that the time constant on the flo history block was increased from 1,000 to 10,000. Additional lim (limit) blocks were introduced to limit the solutions to real values. This program was not capable of generating real values at all possible combinations of this embodiment. However, excellent results were obtained in the multidimensional space occupied by A/D measurement.

In summary, this invention provides a new, effective assay for determining the concentration of an analyte in solution. This assay utilizes analog to digital signaling for determining the concentration of an analyte, employing binding component technology. This novel assay allows quick and simple measurement without complicated machinery or processes. These assays can be administered by employing a hand-held, portable, thermometer-like apparatus.

Having now fully described the invention, it will be apparent to one of ordinary skill in the art that many changes and modifications can be made thereto without departing from the spirit or scope of the invention as set forth herein. It is intended that the invention be limited solely by the lawful scope of the appended claims.

## Claims

1. An assay for signaling the presence of a concentration of analyte in a sample when the sample analyte concentration exceeds a predetermined threshold analyte concentration, comprising

    a quantity of a binding component capable of binding said analyte, said binding component having an analyte dissociation constant substantially less than said threshold analyte concentration, said binding component having a predetermined quantity of empty binding sites, wherein this predetermined quantity of empty binding sites is chosen such that, in the presence of said threshold concentration of analyte, substantially all of said empty binding sites will be filled by substantially all of said analyte in the sample; and

    means for generating a signal when the sample analyte concentration exceed said analyte threshold concentration, said means having an affinity for said analyte which is substantially less than the affinity of the bending component for said analyte.

2. An assay according to claim 1, wherein said binding component is an antibody.

3. An assay according to claim 1, wherein the binding ratio is at least about 1,000.

4. An assay according to claim 3, wherein the binding ratio is greater than about 1,000.

5. An assay according to claim 4, wherein the binding ratio is greater than about 10,000.

6. An assay according to any one of claims 1 to 5, wherein the means for generating a signal when the analyte is at a concentration greater than the threshold comprises a receptor that is a catalyst that undergoes a change in activity in the presence of analyte.

7. An assay according to claim 6, wherein
said receptor is a protein catalyst that undergoes a change in catalytic activity when bound to analyte; and
wherein said analyte is an inhibitor, stimulator, cofactor, or effector of the protein catalyst.

8. An assay according to claim 7, wherein the partition ratio is at least about 10.

9. An assay according to claim 8, wherein the partition ratio is at least about 100.

10. An assay according to claim 9, wherein the partition ratio is at least about 1,000.

11. An assay according to any of claims 1 to 5, wherein
the signal generating means fills a portion of the available binding sites of said binding component; and
the analyte in excess of the threshold concentration displaces said signal generating means from said binding sites resulting in production of a signal.

12. An assay according to claim 11, wherein said signal generating means comprises an analyte-signal conjugate.

13. An assay according to claim 12, wherein said analyte-signal conjugate comprises an analyte-enzyme conjugate, wherein the enzyme of the conjugate is inhibited when bound to said binding component.

14. An assay according to claim 12, wherein said analyte-signal conjugate comprises an analyte-fluorescent conjugate, wherein the fluorescent molecule of the conjugate experiences a detectable change in fluorescent behavior when bound to said binding component versus when it is unbound from said binding component.

15. An assay according to claim 12, wherein the ratio of the affinities of the binding component for analyte to conjugate is greater that about 1.

16. An assay according to claim 15, wherein the affinity constant ratio is greater than about 10.

17. An assay according to any one of claims 1 to 5, wherein
the signal generating means fills a portion of the available binding sites of said binding component;
the analyte in excess of the threshold concentration displaces said signal generating means from said binding sites of the binding component;
said signal generating means is capable of binding with a factor in the free state to form a signal generating means-factor complex when free from said binding component resulting in production of a signal; and
the affinity of said factor for signal generating means is significantly less than the affinity of the binding component for said signal generating means.

18. An assay according to claim 17, wherein said signal generating means comprises an analyte-cofactor conjugate

19. An assay according to claim 18, wherein the ratio of the affinities of the binding component for analyte to conjugate is greater that about 1.

**20.** An assay according to claim 19, wherein the affinity constant ratio is greater that about 10.

**21.** An assay according to claim 18, wherein
said cofactor comprises an enzyme subunit fragment; and
said factor comprises another fragment of said enzyme.

**22.** An assay according to claim 18, wherein
said cofactor comprises a fluorescent molecule; and
said factor comprises a molecule that alters the fluorescence of said cofactor.

**23.** An assay according to claim 18, wherein
said cofactor comprises a molecule that is capable of altering the activity of a catalystic molecule, and
said factor is a catalystic molecule having an altered activity as a result of the cofactor.

**24.** An assay according to claim 23, wherein
said factor is an enzyme of apoenzyme that is inactive in the absence of cofactor; and
said cofactor is a molecule that conveys activity to said enzyme of apoenzyme.

**25.** An assay according to claim 23, wherein
said factor is an apoenzyme that is inactive, but is capable of spontaneously combining with added coenzyme to generate active enzyme, and
said cofactor is a coenzyme-analyte conjugate that is capable of reacting with said apoenzyme when free in solution.

**26.** An assay according to claim 18, wherein said binding component is an antibody.

**27.** An apparatus for performing assays according to claim 1, wherein said apparatus comprises a set of compartments in which each compartment comprises
a binding component that has a predetermined quantity of said empty binding sites; and
signal generating means.

**28.** An apparatus according to claim 27, wherein each compartment comprises
at least one layer of said signal generating means; and
at least one layer of said binding component.

**29.** An apparatus according to claim 27, wherein said binding component is an antibody.

**30.** A method for performing an assay to determine if the concentration of analyte in a sample exceeds a predetermined threshold analyte concentration comprising
A. providing in an assay reaction vessel:
1. a quantity of binding component which binds said analyte, in which said binding component has an analyte dissociation constant substantially less than said threshold analyte concentration, and said binding component has a predetermined quantity of binding sites which have no analyte bound thereto, wherein
the quantity of empty binding sites is chosen such that in the presence of said threshold concentration of analyte, substantially all of said empty binding sites will be filled by substantially all of said analyte in the sample;
2. means for generating a signal when the sample analyte concentration exceeds said analyte threshold concentration, wherein said means has a negligible affinity for said analyte; and
B. adding to said assay vessel a quantity of sample analyte.

**31.** A method according to claim 30, wherein said binding component is an antibody.

# FIG. 1(a)

## TRADITIONAL A/A HOMOGENEOUS ASSAYS

ENERGY

ANALYTE

SIGNAL-GENERATING
STATE

REACTION PATHWAY

# FIG. 1(b)

## A/D ANTIBODY SIGNALING

ENERGY

ANALYTE

SIGNAL-GENERATING
STATE

TIGHT BINDING -
NON-SIGNAL GENERATING

REACTION PATHWAY

## FIG. 2

[ANTIBODY-ANALYTE] x $10^{-10}$ vs LOG [ANALYTE]

## FIG. 3

ANTIBODY-ANALYTE x $10^{-8}$ vs LOG [ANALYTE]

## FIG. 4

## FIG. 7

EP 0 494 509 A1

FIG. 5

A/D SWITCH RESOLUTION AS $F(K_1 / K_2)$

[RECEPTOR] / [ANTIBODY] = 1000

[RECEPTOR-ANALYTE]

$K_1/K_2$

| ANALYTE TOTAL | 1E8 | 1E6 | 1E5 | 1E4 |
|---|---|---|---|---|
| 7.00E-09 | 2.43E-13 | 2.30-11 | 2.10E-10 | 1.27E-09 |
| 8.00E-09 | 4.69E-13 | 3.91-11 | 3.24E-10 | 1.63E-09 |
| 9.00E-09 | 9.80E-13 | 8.09E-11 | 5.31 E-10 | 2.06E-09 |
| 1.00E-08 | 2.99E-11 | 2.97E-10 | 9.05E-10 | 2.55E-09 |
| 1.10E-08 | 9.10E-10 | 9.99E-10 | 1.48E-09 | 3.12E-09 |
| | | | | |
| RESOLUTION | 929 | 12 | 3 | 2 |
| | | | | |
| PARTITION RATIO | 10,000 | 100 | 10 | 1 |

$[ANTIBODY] = 10^{-8}$ M

$[RECEPTOR] = 10^{-5}$ M

$K_2 = 10^6$ M$^{-1}$ THROUGHOUT,

K1 IS VARIED

RESOLUTION IS DEFINED AS THE INCREASE IN SIGNAL SEEN WHEN ANALYTE IS INCREASED FROM 9 nMOLAR TO 11nMOLAR. THE PARTITION RATIO IS DEFINED AS THE RATIO OF BINDING CONSTANTS $(K_1 / K_2)$, DIVIDED BY THE RATIO OF RECEPTOR TO ANTIBODY, SEE TEXT.

**FIG. 6**

1) RANDOM SYNTHESIS

ENZYME

ANTIBODY

INACTIVE

+

ACTIVE

2) DEFINED SYNTHESIS

+

C

INHIBITOR WITH
REACTIVE GROUP

C

ENZYME LABELLED WITH
REACTIVE GROUP (I)

LEAVE AT
SITE C.
DIALYZE
INHIBITOR (II)

+

C          C

ANALYTE

C

ANALYTE

C

+

ANTIBODY

ANALYTE

C - C⁻

INACTIVE ENZYME

EP 0 494 509 A1

| ANTI a2 1 | | SUM 10 | FIO 11 | (x1) | | | |
| --- | --- | --- | --- | --- | --- | --- | --- |

-x3

MUL 12 — LIM 13 — x3 — ADL 14

K1

| ANALYTE a1 2 | SUM 20 | FIO 21 | (x2) |

-x3 -x5

K2 — MUL 32 — LIM 33 — x5 — ADL 34

| RECEPTOR a3 3 | SUM 30 | FIO 31 | (x4) | MUL 32 |

-x5

K1 4

K2 5

**FIG. 8**

```
 1,   CON          ;  ANTIBODY TOTAL
 2,   CON          ;  ANALYTE TOTAL
 3,   CON          ;  RECEPTOR
 4,   CON          ;  K1
 5,   CON          ;  K2
10,   SUM,1,-13    ;  FREE ANTIBODY
11,   FIO,10       ;  ANALOG DELAY
12,   MUL,11,4,21  ;  ANTIBODY-ANALYTE
13,   LIM,12       ;  LIMITS ANTIBODY-ANALYTE TO REAL SOLUTIONS
14,   ADL,13       ;  [ANALYTE-ANTIBODY]
20,   SUM2,-13,-33 ;  FREE ANTIBODY
21,   FIO,20       ;  ANALOG DELAY
30,   SUM,3,-33    ;  FREE RECEPTOR
31,   FIO,30       ;  ANALOG DELAY
32,   MUL,21,31,5  ;  ANALYTE-RECEPTOR
33,   LIM,32       ;  LIMITS ANALYTE-RECEPTOR TO REAL SOLUTIONS
34,   ADL,33       ;  [ANALYTE-RECEPTOR]
```

FIG. 9

| | | |
|---|---|---|
| 1, | CON | ; ANALYTE TOTAL |
| 2, | CON | ; ANTIBODY TOTAL |
| 3, | CON | ; ENZYME TOTAL |
| 4, | CON | ; K1 |
| 5, | CON | ; K2 |
| 10, | SUM,1,-13 | ; FREE ANALYTE |
| 11, | FIO,10 | ; ANALOG DELAY = 1000 |
| 12, | MUL,11,4,21 | ; ANTIBODY-ANALYTE |
| 13, | LIM,12 | ; LIMITS ANTIBODY-ANALYTE TO REAL |
| 14, | ADL,13 | ; [ANTIBODY-ANALYTE] |
| 20, | SUM,2,-13,-23 | ; FREE ANTIBODY |
| 21, | FIO,20 | ; ANALOG DELAY = 1000 |
| 22, | MUL,21,5,31 | ; ANTIBODY-ENZYME |
| 23, | LIM,22 | ; LIMITS ANTIBODY-ENZYME TO REAL |
| 24, | ADL,23 | ; [ANTIBODY-ENZYME] |
| 30, | SUM,3,-23 | ; FREE ENZYME |
| 31, | FIO,30 | ; ANALOG DELAY = 1000 |
| 32, | ADL,31 | ; [ENZYME] |

**FIG. 10(a)**

| | | | |
|---|---|---|---|
| ANALYTE 1 | SUM 10 | FIO 11 | LIM x2 12 |

-X5

| | | | |
|---|---|---|---|
| ANTIBODY 2 | SUM 20 | FIO 21 | LIM x1 22 |

-X5
-X6

| | | | |
|---|---|---|---|
| COFACTOR 3 | SUM 30 | FIO 31 | LIM x3 32 |

-X6
-X7

| | | | |
|---|---|---|---|
| ENZYME 4 | SUM 40 | FIO 41 | LIM x4 42 |

-X7

K1 — MUL 13 — LIM x5 14 — x5 — ADL 15

K2 — MUL 23 — LIM x6 24 — x6 — ADL 25

K3 — MUL 33 — LIM x7 34 — x7 — ADL 35

ADL 43

| K1 5 |
|---|

| K2 6 |
|---|

| K3 7 |
|---|

x1 = [ANTIBODY]
x2 = [ANALYTE]
x3 = [COFACTOR]
x4 = [APOENZYME]
x5 = [ANTIBODY-ANALYTE]
x6 = [ANTIBODY-COFACTOR]
x7 = [HOLOENZYME]

## FIG. 10(b)

| 1  | CON |    |      |     | : ANALYTE TOTAL |
| 2  | CON |    |      |     | : ANTIBODY TOTAL |
| 3  | CON |    |      |     | : COFACTOR TOTAL |
| 4  | CON |    |      |     | : ENZYME TOTAL |
| 5  | CON |    |      |     | : K1 ANTIBODY + ANALYTE |
| 6  | CON |    |      |     | : K2 ANTIBODY + COFACTOR |
| 7  | CON |    |      |     | : K3 ENZYME + COFACTOR |
| 10 | CON | 1  |      | -14 | : ANALYTE (x2) |
| 11 | FIO | 10 |      |     | : ANALOG DELAY = 1000 |
| 12 | LIM | 11 |      |     | : LIMIT [ANALYTE] x (2) |
| 13 | MUL | 12 | 5    | 22  | : ANITBODY-ANALYTE (x5) |
| 14 | LIM | 13 |      |     | : LIMITS x5 TO REAL |
| 15 | ADL | 14 |      |     | : [ANTIBODY-ANALYTE] |
| 20 | SUM | 2  | -14  | -24 | : FREE ANTIBODY (x1) |
| 21 | FIO | 20 |      |     | : ANALOG DELAY = 1000 |
| 22 | LIM | 21 |      |     | : LIMITS x1 TO REAL |
| 23 | MUL | 22 | 6    | 32  | : ANTIBODY-COFACTOR (x6) |
| 24 | LIM | 23 |      |     | : LIMITS x6 TO REAL |
| 25 | ADL | 24 |      |     | : [ANTIBODY-COFACTOR] |
| 30 | SUM | 3  | -24  | -34 | : FREE COFACTOR (x3) |
| 31 | FIO | 30 |      |     | : ANALOG DELAY = 1000 |
| 32 | LIM | 31 |      |     | : LIMITS x3 TO REAL |
| 33 | MUL | 32 | 7    | 42  | HOLOENZYME (x7) |
| 34 | LIM | 33 |      |     | : LIMITS x7 TO REAL |
| 35 | ADL | 34 |      |     | : [HOLOENZYME] |
| 40 | SUM | 4  |      | -34 | : APOENZYME (x4) |
| 41 | FIO | 40 |      |     | : ANALOG DELAY = 1000 |
| 42 | LIM | 41 |      |     | : LIMITS x4 TO REAL |

## FIG. 11(a)

SIGNAL STRENGTH AS F(K1/K2)

$K_1 / K_2 = 1$

FREE ENZYME x 10⁻⁹M

[ANALYTE] X 10⁻⁷M

## FIG. 11(b)

$K_1 / K_2 = 100$

FREE ENZYME x 10⁻⁸M

[ANALYTE] X 10⁻⁷M

FIG. 12

CONDITIONS

| CASE = | 1 | 2 | 3 | 4 | 5 | 6 |
|---|---|---|---|---|---|---|
| [ANTIBODY] = | 1.00E-07 | 1.00E-07 | 1.00E-07 | 1.00E-07 | 1.00E-07 | 1.00E-07 |
| [ENZYME] = | 1.00E-08 | 1.00E-09 | 5.00E-08 | 1.00E-08 | 1.00E-08 | 1.00E-08 |
| K1 = | 1.00E+12 | 1.00E+12 | 1.00E+12 | 1.00E+14 | 1.00E+11 | 1.00E+10 |
| K2 = | 1.00E+12 | 1.00E+12 | 1.00E+12 | 1.00E+12 | 1.00E+10 | 1.00E+09 |

FREE ENZYME CONCENTRATION

CASE

| [ANALYTE] | 1 | 2 | 3 | 4 | 5 | 6 |
|---|---|---|---|---|---|---|
| 2.00E-08 | | | 1.7E-12 | | | |
| 3.00E-08 | | | 2.5E-12 | | | |
| 4.00E-08 | | | 5.0E-12 | | | |
| 4.50E-08 | | | 1.0E-11 | | | |
| 5.00E-08 | | | 1.6E-10 | | | |
| 5.50E-08 | | | 2.4E-09 | | | |
| 6.00E-08 | 3.3E-13 | 2.6E-14 | 4.6E-09 | 3.4E-13 | 3.3E-11 | 3.2E-10 |
| 7.00E-08 | 5.0E-13 | 3.5E-14 | 8.4E-09 | 5.0E-13 | 5.0E-11 | 4.6E-10 |
| 8.00E-08 | 1.0E-12 | 5.4E-14 | 1.2E-08 | 1.0E-12 | 9.7E-11 | 8.0E-10 |
| 8.50E-08 | 2.0E-12 | | | 2.0E-12 | 1.8E-10 | 1.2E-09 |
| 9.00E-08 | 3.2E-11 | 1.1E-13 | | 9.5E-11 | 6.9E-10 | 2.0E-09 |
| 9.50E-08 | 4.8E-10 | 2.5E-13 | | 1.2E-09 | 2.4E-09 | 3.1E-09 |
| 1.00E-07 | 9.1E-10 | 1.1E-11 | 1.7E-08 | 1.0E-08 | 4.0E-09 | |
| 1.05E-07 | 1.0E-09 | 5.4E-11 | | 1.0E-08 | | |
| 1.10E-07 | 1.0E-09 | 8.8E-11 | | | 5.9E-09 | |
| 1.15E-07 | | 1.1E-10 | | | | |
| 1.20E-07 | 1.1E-09 | 1.3E-10 | 1.7E-08 | | | |
| 1.30E-07 | | 1.6E-10 | | | | |
| 1.40E-07 | 1.1E-09 | 1.8E-10 | | | | |

| SWITCH RESOLUTION | 240 | 216 | 240 | 600 | 13 | 2.6 |

ANTIBODY + ANALYTE $\xrightarrow{K1}$ ANTIBODY-ANALYTE

ANTIBODY + ENZYME $\xrightarrow{K2}$ ANTIBODY-ENZYME

SWITCH RESOLUTION IS CALCULATED AS THE INCREASE IN SIGNAL STRENGTH OBSERVED WHEN ANALYTE CONCENTRATION IS INCREASED FROM $5 \times 10^{-9}$M BELOW THE THRESHOLD TO $5 \times 10^{-9}$M ABOVE THE THRESHOLD.

**European Patent Office**

# EUROPEAN SEARCH REPORT

Application Number

EP 91 31 1347

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 378 391 (BIOSITE DIAGNOSTICS INC.) <br> * page 1 - page 5; claims * <br> --- | 1-6 | G01N33/53 <br> G01N33/542 |
| A | EP-A-0 327 843 (HYGEIA SCIENCES, INC.) <br> * abstract; claims * <br> --- | 1,2 | |
| A | WO-A-8 606 170 (IMMUNICON CORPORATION) <br><br> * page 1 - page 17; claims * <br> --- | 1,2,6, <br> 12,13 | |
| A,D | US-A-4 442 204 (A.C.GREENQUIST ET AL.) <br><br> ----- | | |
| | | | TECHNICAL FIELDS SEARCHED (Int. Cl.5) |
| | | | G01N |

The present search report has been drawn up for all claims

| Place of search | Date of completion of the search | Examiner |
|---|---|---|
| THE HAGUE | 30 MARCH 1992 | HITCHEN C.E. |